# EUROPEAN PATENT APPLICATION

(11) **EP 4 410 975 A1**
(43) Date of publication of application: **07.08.2024**
(21) Application number: 22876437.9
(22) Date of filing: 29.09.2022
(51) Int. Cl.: C12N 15/09, C12N 5/10

(54) **METHOD FOR PRODUCING CELLS HAVING DNA DELETION SPECIFIC TO ONE OF HOMOLOGOUS CHROMOSOMES**

(30) Priority: 30.09.2021 JP 2021161312
(71) Applicant: OSAKA UNIVERSITY, Suita-shi Osaka 565-0871 (JP)
(72) Inventor: NAKADA Shinichiro, Suita-shi, Osaka 565-0871 (JP); TOMITA Akiko, Suita-shi, Osaka 565-0871 (JP)
(74) Representative: J A Kemp LLP
(86) International application number: PCT/JP2022/036403
(87) International publication number: WO 2023/054573

(57) **Abstract**

It has been found that by generating multiple nicks in the DNA region proximal to a specific site of homologous chromosomes, DNA exceeding 100 bp can be deleted in one of the homologous chromosomes.

## Description

### [Technical Field]

The present invention relates to a method for producing a cell having a DNA deletion specific to one of the homologous chromosomes using a site-specific nickase. The present invention also relates to a method for evaluating homologous recombination function in a cell using the DNA deletion as an indicator.

### [Background Art]

with the advent of programmable nucleases such as the TALENs and CRISPR-Cas systems, genome editing technology is now rapidly expanding. A Cas protein forms a complex with a guide RNA, and this complex binds to a target site on the genome that has a sequence complementary to the guide RNA and cleaves both of the DNA duplex. TALENs, a genome editing technique one generation ago, are a fusion protein of a TALE protein that targets DNA and a nuclease that cleaves DNA (mainly FokI), and cause a double-strand break in DNA at the target site on the genome, similar to the CRISPR-Cas systems.

When this double-strand break of DNA is repaired by non-homologous end joining, nucleotide insertion/deletion (indel) takes place, and gene knockout can be performed by frame shift or the like. Meanwhile, when donor DNA serving as a repair template is introduced from outside the cell, gene knock-in can be performed by homologous recombination between the genome and the donor DNA. In this gene knock-in, it is possible not only to insert DNA but also to cause substitution or deletion of one to several nucleotides.

However, from the viewpoint of genome homeostasis, there are serious problems in genome editing using programmable nucleases. One of them is the generation of unintended gene mutations due to double-strand breaks in DNA. In somatic cells, repair by non-homologous end joining is superior to repair by homologous recombination, and thus unintended mutations (indels) are more likely to take place at target sites that have been subjected to double-strand break by a genome editing system than knock-in with a repair template. Therefore, a cell population subjected to genome editing includes not a few cells with unintended mutations due to non-homologous end joining, in addition to the cells that have achieved the desired knock-in and the cells in which no change has taken place in the gene sequence at all. Also, at the one-cell level, even when one of the autosomal alleles is knocked in as planned, the other may have an unintended mutation. It has also been reported that programmable nucleases cause DNA double-strand breaks in DNA sequences that are highly similar to the target sequence (off-targets), resulting in genomic mutations.

In view of the above, the present inventors have developed methods that can, by using nickase-type Cas9 with one of the two nuclease sites of Cas9 inactivated, induce homologous recombination by single-strand break in DNA to suppress the occurrence of unintended mutations (indels) due to non-homologous end joining as compared with the conventional method by double-strand break in DNA. One of them is a genome editing method that utilizes a tandem nick method by using nickases to nick two sites in the target genome and one site in the donor plasmid containing the repair template (NPL 1 and PTL 1). In addition, the present inventors have further advanced this method to also develop an SNGD method that nicks one site in the target genome and nicks one site in the donor plasmid containing the repair template (a combination of single nicks in the target gene and donor plasmid) (PTL 1).

On the other hand, in genome editing, there is also a problem of random integration of donor DNA used as a repair template into the genome. Therefore, the present inventors have also developed a method to perform genome editing using a homologous chromosome originally present in a cell as a repair template (PTL 2). According to this genome editing, when there is a heterozygous mutation or a compound heterozygous mutation in the chromosome in a cell, by inducing homologous recombination between homologous chromosomes, it is possible to repair the mutation present in one of the homologous chromosomes to a normal sequence using the other homologous chromosome without the mutation as a repair template.

Among the heterozygous mutations present in the chromosomes, for the purpose of treating diseases where the genetic product from the mutant allele inhibits the function of the genetic product from the normal allele, in addition to repair to the normal sequence by such homologous recombination, it is also conceivable to specifically delete the DNA region containing these mutations.

Therefore, there has been a demand to develop a method capable of efficiently deleting a specific DNA region from one of the homologous chromosomes while suppressing occurrence of unintended mutations.

### [Citation List]

### [Patent Literature]

[PTL 1] Japanese Patent Application Publication No. 2018-11525
[PTL 2] International Publication No. WO2020/100361 [Non Patent Literature]
[NPL 1] Nakajima K, et al., Genome Res. 28, 223-230, 2018

### [Summary of Invention]

### [Technical Problem]

The present invention has been made in view of such circumstances, and an object thereof is to provide a method for efficiently causing a DNA deletion specific to one of the homologous chromosomes without introducing double-strand breaks in DNA and without using exogenous donor DNA. Another object of the present invention is to provide a method for evaluating homologous recombination function in a cell using the DNA deletion as an indicator.

### [Solution to Problem]

When there is a heterozygous mutation in the gene of a cell, the genetic mutation existing in one homologous chromosome (hereinafter referred to as "chromosome A") does not exist in the other homologous chromosome (hereinafter referred to as "chromosome B"). Here, if the DNA region containing the mutation in chromosome A can be specifically deleted, it becomes possible to treat diseases caused by this mutation.

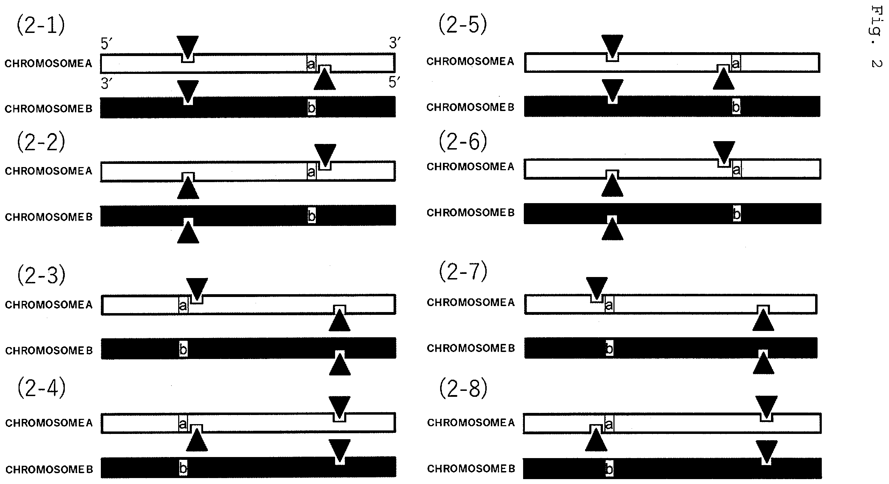

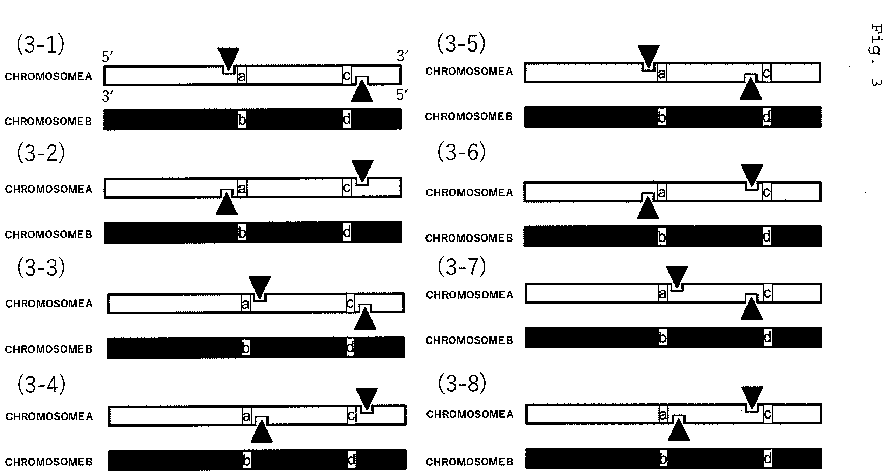

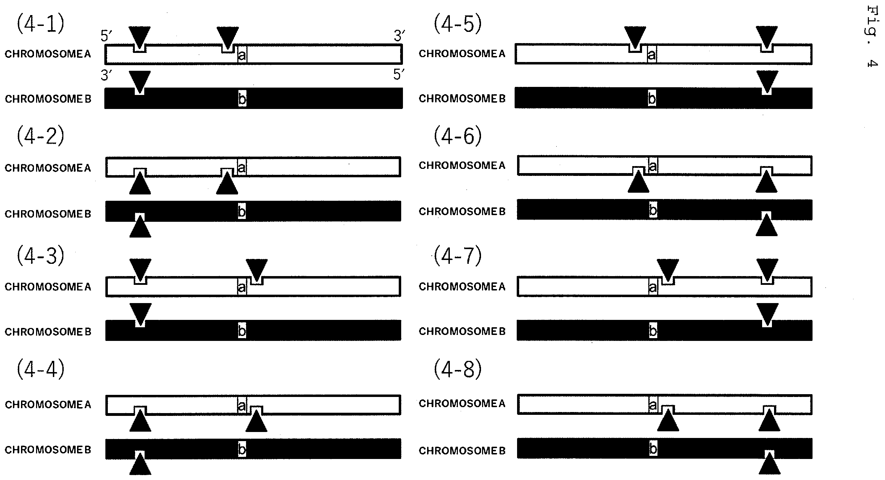

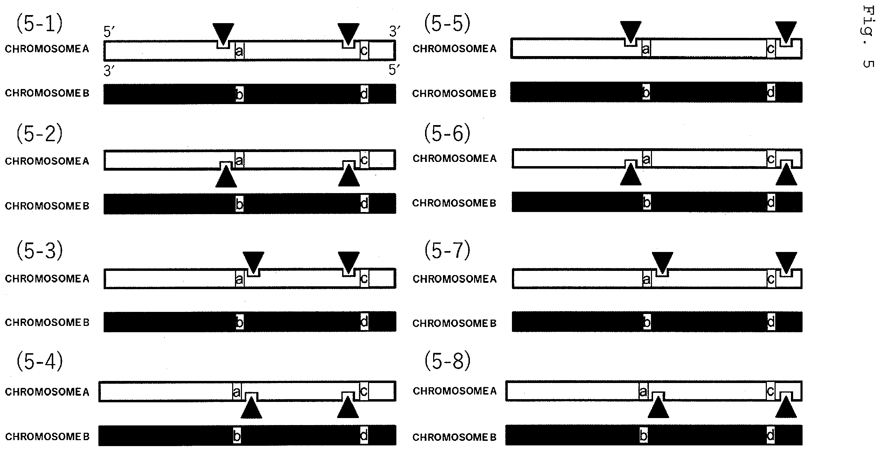

The present inventors have introduced DNA single-strand breaks in various manners around mutation sites in homologous chromosomes and found as a result that it is possible to efficiently delete a DNA region exceeding 100 bp from chromosome A by generating nicks on both DNA strands or a single DNA strand at multiple sites in the DNA region proximal to the mutation site on chromosome A and, on chromosome B, generating a nick at one of the sites corresponding to the sites nicked on chromosome A (an example of introduction of nicks in this embodiment is shown in Figs. 2 and 4) . The present inventors have also found that it is possible to efficiently delete a DNA region exceeding 100 bp from chromosome A even when no nick is generated on chromosome B in the above embodiment (an example of introduction of nicks in this embodiment is shown in Figs. 3 and 5). Furthermore, the present inventors have found that the efficiency of deleting DNA regions can be improved by using cells in which the function of genes involved in homologous recombination (e.g., each FANC gene) is suppressed. -

According to the principle of the method of the present invention, it is possible to widely target differences in bases between homologous chromosomes and delete DNA exceeding 100 bp only from one of the homologous chromosomes. In addition, the fact that suppression of homologous recombination function in cells improved the efficiency of DNA deletion suggests that it is possible to evaluate homologous recombination function in cells using the DNA deletion as an indicator.

The present invention is based on these findings, and more specifically provides the following:
(1) A method for producing a cell having a DNA deletion specific to chromosome A of homologous chromosomes consisting of chromosome A and chromosome B, comprising:
   introducing, into a cell having different bases between homologous chromosomes at a specific site of the homologous chromosomes, a combination of site-specific nickases each of which causes a single-strand break at a DNA region proximal to the specific site, wherein
   the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites.
(2) The method according to (1), wherein the cell having different bases between homologous chromosomes at a specific site of the homologous chromosomes is a cell with suppressed function of a FANC gene.
(3) The method according to (1) or (2), wherein the bases different between homologous chromosomes are bases at a mutation site.
(4) The method according to any one of (1) to (3), wherein each of the site-specific nickases is a CRISPR-Cas system.
(5) A kit for use in the method according to (1), comprising:
   in a cell having different bases between homologous chromosomes at a specific site of the homologous chromosomes, a combination of site-specific nickases each of which causes a single-strand break at a DNA region proximal to the specific site, wherein
   the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites.
(6) The kit according to (5), wherein the cell having different bases between homologous chromosomes at a specific site of the homologous chromosomes is a cell with suppressed function of a FANC gene.
(7) A method for evaluating homologous recombination function in a cell, comprising:
   introducing, into a cell having different bases between homologous chromosomes at a specific site of the homologous chromosomes consisting of chromosome A and chromosome B, a combination of site-specific nickases each of which causes a single-strand break at a DNA region proximal to the specific site; and detecting chromosome A-specific DNA deletion generated thereby, wherein
   the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites, and
   the homologous recombination function in a cell is evaluated as suppressed when a frequency of chromosome A-specific DNA deletion occurring is higher than a control.
(8) The method according to (7), wherein the cell is a cancer cell.
(9) The method according to (7) or (8), wherein each of the site-specific nickases is a CRISPR-Cas system.
(10) A kit for use in the method according to (7), comprising:
   in a cell having different bases between homologous chromosomes at a specific site of the homologous chromosomes consisting of chromosome A and chromosome B, a combination of site-specific nickases each of which causes a single-strand break at a DNA region proximal to the specific site, wherein
   the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites.

### [Advantageous Effects of Invention]

According to the present invention, it is possible to efficiently cause a DNA deletion specific to one of the homologous chromosomes. In the method of the present invention, since double-strand breaks are not introduced in DNA, unintended mutations are unlikely to occur throughout the genome including chromosome B. In addition, since exogenous donor DNA is not used, there is no problem of random integration of donor DNA. Therefore, even when the present invention is applied to medicine such as gene therapy, the safety is high. Further, according to the present invention, it is possible to evaluate the homologous recombination function in a cell using the DNA deletion specific to one of the homologous chromosomes as an indicator.

### [Brief Description of Drawings]

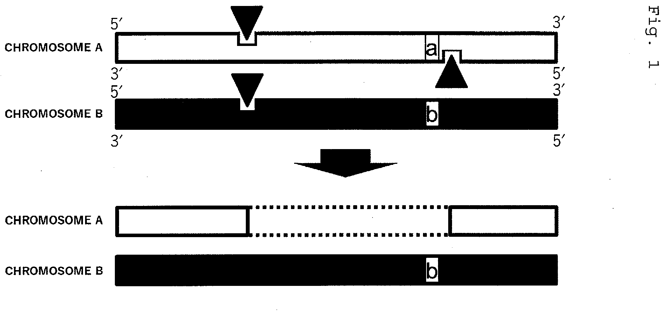

[Fig. 1] Fig. 1 is a conceptual diagram (one example) of the method of the present invention. The bases at target site a on chromosome A and target site b on chro mosome B are different bases. In this example, a nick is generated at one location on each DNA strand in the vicinity of target site a on chromosome A, and a nick i s generated at a location corresponding to one of the 1 ocations where nicks are generated on chromosome A. Th is results in deletion of a DNA fragment on chromosome A.
[Fig. 2] Fig. 2 is a diagram showing an example of an embodiment in the method of the present invention (firs t embodiment of the present invention) in which a nick is generated at one location on each DNA strand in the vicinity of target site a on chromosome A and a nick is generated at a location corresponding to one of the lo cations where nicks are generated on chromosome A.
[Fig. 3] Fig. 3 is a diagram showing an example of an embodiment in the method of the present invention (seco nd embodiment of the present invention) in which a nick is generated at one location on each DNA strand in the vicinity of target site a on chromosome A and no nick is generated at locations corresponding to the location s where nicks are generated on chromosome A.
[Fig. 4] Fig. 4 is a diagram showing an example of an embodiment in the method of the present invention (thir d embodiment of the present invention) in which two nic ks are generated on the same DNA strand in the vicinity of target site a on chromosome A and a nick is generat ed at a location corresponding to one of the locations where nicks are generated on chromosome A.
[Fig. 5] Fig. 5 is a diagram showing an example of an embodiment in the method of the present invention (four th embodiment of the present invention) in which two ni cks are generated on the same DNA strand in the vicinit y of target site a on chromosome A and no nick is gener ated at locations corresponding to the locations where nicks are generated on chromosome A.

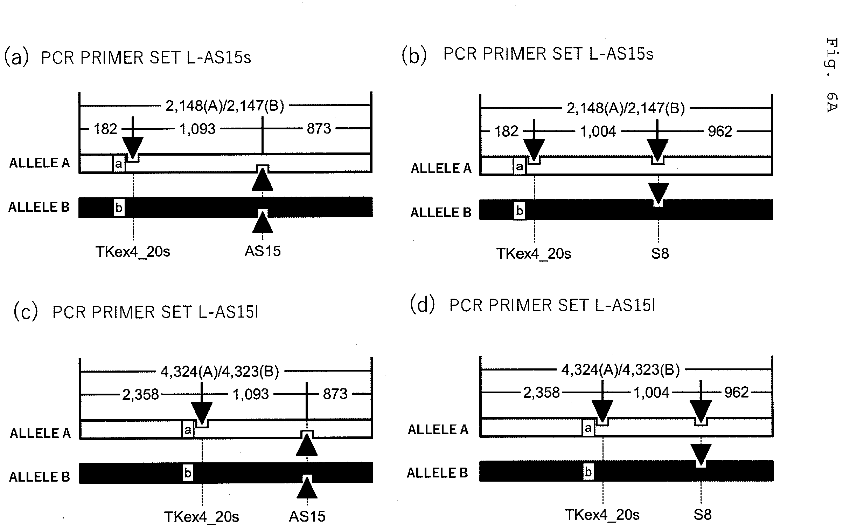

[Fig. 6A] Fig. 6A is a diagram showing the positional relationship between PCR products obtained using PCR p rimer set L-AS15s or PCR primer set L-AS15l and nicks. (a) shows PCR primer set L-AS15s and nicks by sgRNA TK ex4_20s and sgRNA AS15; (b) shows PCR primer set L-AS15 s and nicks by sgRNA TKex4_20s and sgRNA S8; (c) shows PCR primer set L-AS15l and nicks by sgRNA TKex4_20s and sgRNA AS15; (d) shows PCR primer set L-AS15l and nicks by sgRNA TKex4_20s and sgRNA S8. The nicks in (a) and (c) are examples of the embodiment shown in Fig. 2 (fi rst embodiment of the present invention), and the nicks in (b) and (d) are examples of the embodiment shown in Fig. 4 (third embodiment of the present invention).

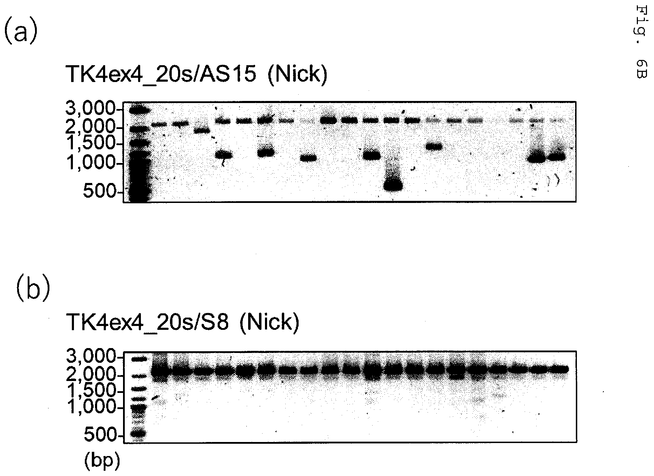

[Fig. 6B] Fig. 6B is an electrophoretic image when a cell clone with DNA deletion was identified using PCR p rimer set L-AS15s. (a) shows the result of introducing nicks with sgRNA TKex4_20s and sgRNA AS15; (b) shows t he result of introducing nicks with sgRNA TKex4_20s and sgRNA S8.

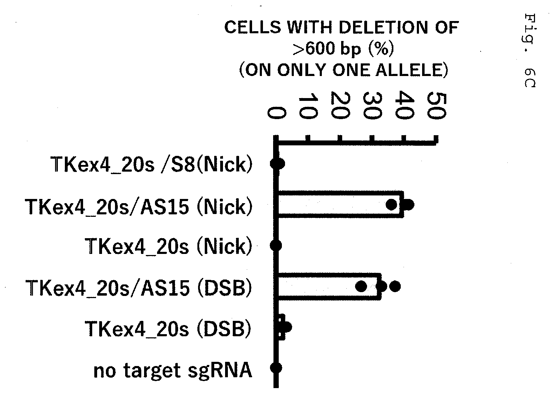

[Fig. 6C] Fig. 6C is a graph showing the percentage o f cell clones in which a deletion of 600 bp or more was detected in only one of the homologous chromosomes. N inety-four clones were used in each experiment, and the experiment was performed in triplicate.

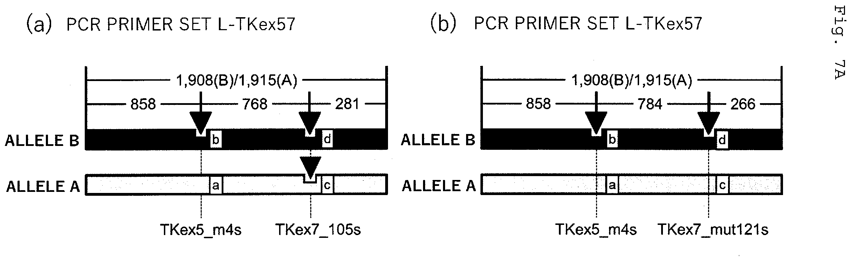

[Fig. 7A] Fig. 7A is a diagram showing the positional relationship between PCR products obtained using PCR p rimer set L-TKex57 and nicks. (a) shows PCR primer set L-TKex57 and nicks by sgRNA TKex5_m4s and sgRNA TKex7_ 105s; (b) shows PCR primer set L-TKex57 and nicks by sg RNA TKex5_m4s and sgRNA TKex7_mut121s. The nicks in (a ) are an example of the embodiment shown in Fig. 4 (thi rd embodiment of the present invention), and the nicks in (b) are an example of the embodiment shown in Fig. 5 (fourth embodiment of the present invention).

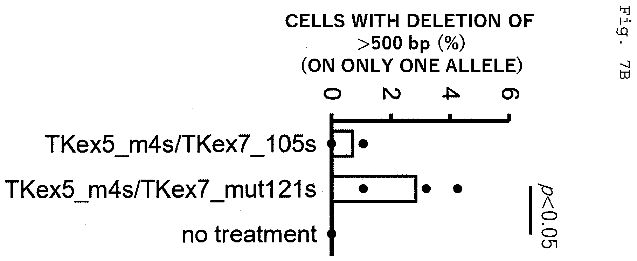

[Fig. 7B] Fig. 7B is a graph showing the percentage o f cell clones in which a deletion of 500 bp or more was detected in only one of the homologous chromosomes. N inety-four clones were used in each experiment, and the experiment was performed in triplicate.

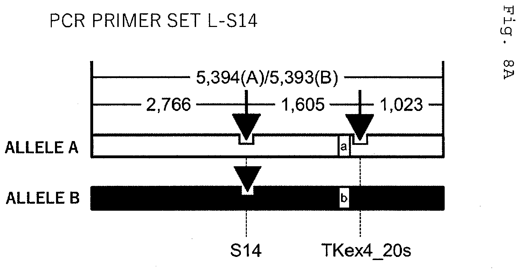

[Fig. 8A] Fig. 8A is a diagram showing the positional relationship between PCR products obtained using PCR p rimer set L-S14 and nicks. It shows PCR primer set L-S 14 and nicks by sgRNA S14 and sgRNA TKex4_20s. The nic ks in this figure are an example of the embodiment show n in Fig. 4 (third embodiment of the present invention)

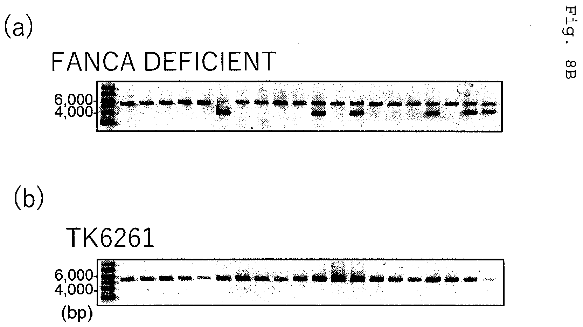

[Fig. 8B] Fig. 8B is an electrophoretic image when a cell clone with DNA deletion was identified using PCR p rimer set L-S14. (a) shows the result of introducing n icks with sgRNA TKex4_20s and sgRNA S14 in FANCA mutant cells; (b) shows the result of introducing nicks with sgRNA TKex4_20s and sgRNA S14 in the parent cell line T K6261.

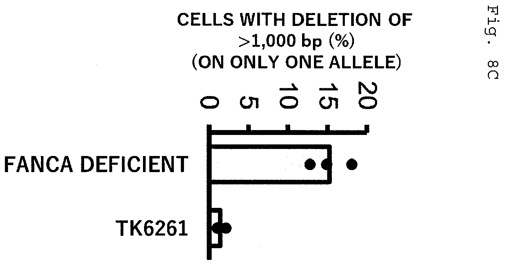

[Fig. 8C] Fig. 8C is a graph showing the percentage o f cell clones in which a deletion of 1000 bp or more wa s detected in only one of the homologous chromosomes. Ninety-four clones were used in each experiment, and th e experiment was performed in triplicate.

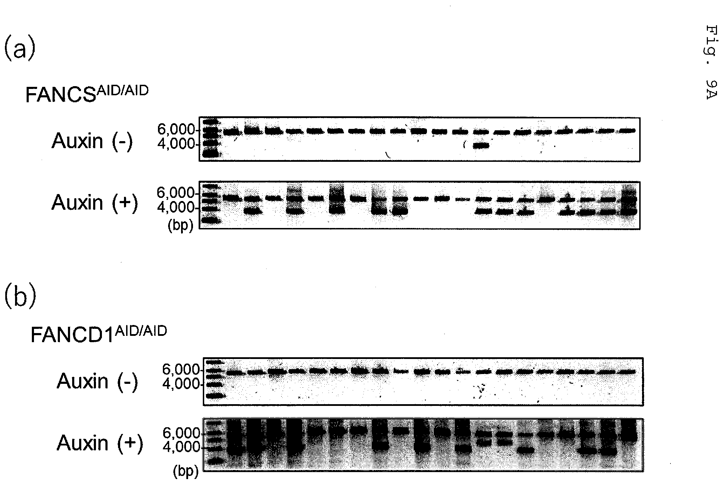

[Fig. 9A] Fig. 9A is an electrophoretic image when a cell clone with DNA deletion was identified using PCR p rimer set L-S14. The upper part of (a) shows the resul t of introducing nicks with sgRNA TKex4_20s and sgRNA S 14 in cells in which FANCS protein was depleted by auxi n induction; the lower part thereof shows the result in cells without auxin induction (control). The upper pa rt of (b) shows the result of introducing nicks with sg RNA TKex4_20s and sgRNA S14 in cells in which FANCD1 pr otein was depleted by auxin induction; the lower part t hereof shows the result in cells without auxin inductio n (control).

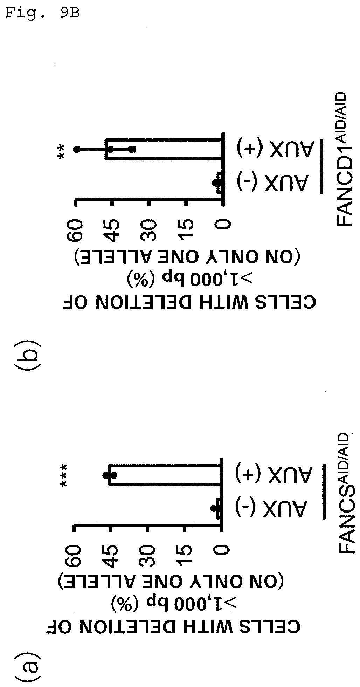

[Fig. 9B] Fig. 9B is a graph showing the percentage o f cell clones in which a deletion of 1000 bp or more wa s detected in only one of the homologous chromosomes. (a) shows the results in cells in which FANCS protein w as depleted by auxin induction (AUX(+)) or cells withou t auxin induction (AUX(-)) (control). (b) shows the re sults in cells in which FANCD1 protein was depleted by auxin induction (AUX(+)) or cells without auxin inducti on (AUX(-)) (control). Ninety-four clones were used in each experiment, and the experiment was performed in t riplicate.

### [Description of Embodiments]

### 1. Method and kit for producing a cell having DNA deletion

The present invention provides a method for producing a cell having a DNA deletion specific to chromosome A of homologous chromosomes consisting of chromosome A and chromosome B.

The method of the present invention comprises introducing, into a cell having different bases between homologous chromosomes at a specific site of the homologous chromosomes, a combination of site-specific nickases each of which causes a single-strand break at a DNA region proximal to the specific site.

In the present invention, the "different bases between homologous chromosomes" at a specific site of the homologous chromosomes may be one base or multiple bases (base sequence). Moreover, it may be a mutation or a polymorphism. Examples of mutations include substitutions, deletions, insertions, or combinations thereof, and examples of polymorphism include single base polymorphism and microsatellite polymorphism. Note that the base may be present in the genetic coding region or in the genetic non-coding region.

In the present invention, if one of the homologous chromosomes has a mutation or polymorphism at a specific site thereof, it is also possible to cause deletion of DNA containing that mutation or polymorphism in the chromosome, or to cause a corresponding DNA deletion in the other chromosome.

A typical use aspect of the present invention from the viewpoint of medical usefulness is, in order to treat or prevent human diseases caused by heterozygous mutations, to delete DNA containing those mutations in human cells. Preferable examples of heterozygous mutations to be treated or prevented include gain-of-function mutations and dominant negative mutations. Here, examples of the "diseases caused by heterozygous mutations" include, but are not limited to, diseases caused by autosomal heterozygous mutations inherited in an autosomal dominant manner (e.g., congenital immunodeficiency disorders such as OAS1 abnormality, severe congenital neutropenia due to ELANE mutation, chronic mucocutaneous candidiasis, hereditary motor, sensory, and autonomic neuropathy type IIC, post-synaptic slow-channel congenital myasthenic syndrome, Parkinson's disease type 8, tubular aggregate myopathy, thyroid hormone resistance, hyper-IgE syndrome, Ehlers-Danlos syndrome, collagen disorders (such as osteogenesis imperfecta, dystrophic epidermolysis bullosa, and type II collagen disorder), retinal pigmentary degeneration, achondrogenesis, etc.), and triplet repeat diseases exhibiting autosomal dominant inheritance (e.g., Huntington's disease, spinocerebellar ataxia, myotonic dystrophy, Friedreich's ataxia, oculopharyngeal muscular dystrophy, etc.).

The "site-specific nickase" used in the present invention is not limited as long as it can cleave a single strand of DNA in a site-specific manner on the genome, but is preferably a CRISPR-Cas system having a nickase-type Cas protein as a component. Cas proteins typically include a domain involved in cleavage of the target strand (RuvC domain) and a domain involved in cleavage of the non-target strand (HNH domain), whereas the nickase-type Cas protein typically loses its cleavage activity due to a mutation in either of these two domains. In the case of spCas9 protein (Cas9 protein derived from S. pyogenes), examples of such mutation include a mutation of the 10th amino acid (aspartic acid) from the N-terminus to alanine (D10A: mutation within the RuvC domain), a mutation of the 840th amino acid (histidine) from the N-terminus to alanine (H840A: mutation within the HNH domain), a mutation of the 863th amino acid (asparagine) from the N-terminus to alanine (N863A: mutation within the HNH domain), a mutation of the 762th amino acid (glutamic acid) from the N-terminus to alanine (E762A: mutation within the RuvCII domain), and a mutation of the 986th amino acid (aspartic acid) from the N-terminus to alanine (D986A: mutation within the RuvCIII domain). In addition, Cas9 proteins of various origins are known (for example, WO2014/131833), and their nickase types can be utilized. Note that amino acid sequences and base sequences of Cas9 proteins are registered in a public database, for example, GenBank (http://www.ncbi.nlm.nih.gov) (for example, accession number: Q99ZW2.1 and the like), and these can be used in the present invention.

Further, in the present invention, it is also possible to use Cas proteins other than Cas9, such as Cpf1 (Cas12a), Cas12b, CasX (Cas12e), and Cas14. Examples of mutations in the nickase-type Cpf1 proteins include, in AsCpf1 (Cas12), a mutation of the 1226th amino acid (arginine) from the N-terminus to alanine (R1226A: mutation within the Nuc domain). Amino acid sequences of Cpf1 are registered in a public database, for example, GenBank (http://www.ncbi.nlm.nih.gov) (for example, accession numbers: WP_021736722, WP_035635841, and the like).

As the protein constituting the CRISPR-Cas system, for example, a protein added with a nuclear localization signal may be used.

In the CRISPR-Cas system including a nickase-type Cas protein as a component, the nickase-type Cas protein binds to a guide RNA to form a complex, and is targeted to a target DNA sequence to cleave a single strand of DNA. In the CRISPR-Cas9 system, the guide RNA includes crRNA and tracrRNA, but in the CRISPR-Cpfl system, tracrRNA is unnecessary. The guide RNA in the CRISPR-Cas9 system may be a single-molecule guide RNA containing crRNA and tracrRNA, or a double-molecule guide RNA composed of a crRNA fragment and a tracrRNA fragment.

The crRNA contains a base sequence complementary to the target DNA sequence. The target DNA sequence is a base sequence composed of usually 12 to 50 bases, preferably 17 to 30 bases, and more preferably 17 to 25 bases, and is preferably selected from regions adjacent to the PAM (proto-spacer adjacent motif) sequence. Typically, site-specific cleavage of DNA takes place at positions determined by both the complementarity of base pairing between the crRNA and the target DNA sequence and the PAM present adjacent thereto.

In many CRISPR-Cas systems, crRNA also contains a base sequence on the 3'-side that can interact (hybridize) with the tracrRNA fragment. Meanwhile, tracrRNA contains a base sequence that can interact (hybridize) with a part of the base sequence of crRNA on the 5'-side. By the interaction of these base sequences, crRNA/tracrRNA (one molecule or two molecules) form a double-stranded RNA, and the formed double-stranded RNA interacts with the Cas protein.

PAM varies depending on the type and origin of Cas protein. Typical PAM sequences are, for example, "5'-NGG" for Cas9 protein (type II) derived from S. pyogenes, "5'-CCN" for Cas9 protein (type I-A1) derived from S. solfataricus, "5'-TCN" for Cas9 protein (type I-A2) derived from S. solfataricus, "5'-TTC" for Cas9 protein (type I-B) derived from H. walsbyi, "5'-AWG" for Cas9 protein (type I-E) derived from E. coli, "5'-CC" for Cas9 protein (type I-F) derived from E. coli, "5'-CC" for Cas9 protein (type I-F) derived from P. aeruginosa, "5'-NNAGAA" for Cas9 protein (type II-A) derived from S. Thermophilus, "5'-NGG" for Cas9 protein (type II-A) derived from S. agalactiae, "5'-NGRRT" or "5'-NGRRN" for Cas9 protein derived from S. aureus, "5'- NNNNGATT" for Cas9 protein derived from N. meningitidis, and "5'-NAAAAC" for Cas9 protein derived from T. denticola. In Cpfl, it is typically "5'-TTN" or "5'-TTTN." Note that it is also possible to modify PAM recognition by modifying the protein (for example, introducing a mutation) (Benjamin, P. et al., Nature 523, 481-485 (2015), Hirano, S. et al., Molecular Cell 61, 886-894 (2016), and Walton, R. T. et al., Science 368, 290-296 (2020)) .

In the present invention, a site-specific nickase other than CRISPR-Cas systems can also be used. Examples of such site-specific nickase include an artificial nuclease fused with an enzyme having nickase activity. Examples of artificial nucleases usable include TALE (transcription activator-like effector), ZF (zinc finger), and PPR (pentatricopeptide repeat). Examples of enzymes capable of exerting nickase activity by fusion with these artificial nucleases include TevI (Nat Commun. 2013; 4: 1762. doi: 10.1038/ncomms2782) . These artificial nucleases are targeted to the target DNA sequence by a DNA-binding domain constructed by linking modules (peptides) that recognize specific bases (or specific base sequences), and cleave a single strand of DNA with a nickase fused to the DNA-binding domain. A suitable spacer peptide may be introduced between the DNA binding domain and the nickase in an artificial nuclease.

In the present invention, single-strand breaks are generated at multiple sites in the DNA region proximal to a specific site (i.e., different bases between homologous chromosomes) in one of the homologous chromosomes (i.e., chromosome A). In one embodiment, both DNA strands in the proximal DNA region are single-strand broken, and in another embodiment, multiple sites on the same DNA strand in the proximal DNA region are single-strand broken. Also, in the present invention, in the other homologous chromosome (i.e., chromosome B), either a site corresponding to one of the sites single-strand broken in chromosome A (hereinafter simply referred to as "corresponding site") is single-strand broken, or no single-strand break is generated at the corresponding site. Therefore, the present invention includes the following embodiments:
First embodiment: In chromosome A, both DNA strands in the proximal DNA region are single-strand broken, and in chromosome B, one of the corresponding sites is single-strand broken (Fig. 2).
Second embodiment: In chromosome A, both DNA strands in the proximal DNA region are single-strand broken, and in chromosome B, no single-strand break occurs at the corresponding site (Fig. 3).
Third embodiment: In chromosome A, multiple sites on the same DNA strand in the proximal DNA region are single-strand broken, and in chromosome B, one of the corresponding sites is single-strand broken (Fig. 4).
Fourth embodiment: In chromosome A, multiple sites on the same DNA strand in the proximal DNA region are single-strand broken, and in chromosome B, no single-strand break occurs at the corresponding site (Fig. 5).

Here, "proximal DNA region" means a region within normally 100000 bases (e.g., within 10000 bases, within 5000 bases, within 2000 bases, within 1000 bases, or within 600 bases) from the specific site. The distance between the introduced nicks is normally 100 bases or more (e.g., 200 bases or more, 300 bases or more, 500 bases or more, 700 bases or more, 1000 bases or more, or 1500 bases or more). It is preferable that one of the nicks introduced into the chromosome is immediately proximal to the specific site. Here, "immediately proximal" means normally within 100 bases, and more preferably within 50 bases (e.g., within 40 bases, within 30 bases, within 20 bases, or within 10 bases).

Specific examples include an embodiment in which a single-strand break is generated at one location on each DNA strand sandwiching the specific site in chromosome A (2-1, 2-2, 2-7, and 2-8 in Fig. 2), an embodiment in which a single-strand break is generated at each of both locations on the same DNA strand sandwiching the specific site (4-3 to 4-6 in Fig. 4), an embodiment in which a single-strand break is generated at one location on each DNA strand on one side of the specific site (2-3 to 2-6 in Fig. 2), and an embodiment in which two single-strand breaks are generated on the same DNA strand on one side of the specific site (4-1, 4-2, 4-7, and 4-8 in Fig. 4). In addition, another specific site (different bases between homologous chromosomes) may exist in the DNA region proximal to one specific site (Figs. 3 and 5). In this case, if nicks are introduced to sandwich multiple specific sites, DNA containing multiple specific sites can be deleted in chromosome A. In addition, in chromosome A, the number of sites to be single-strand broken may be three or more.

When simultaneously single-strand breaking corresponding sites in two chromosomes constituting homologous chromosomes (i.e., chromosome A and chromosome B), site-specific nickases that bind to the target DNA sequence in chromosome A may be designed to also bind to the corresponding DNA sequence in chromosome B. In this case, the target DNA sequence in chromosome A and the corresponding DNA sequence in chromosome B are typically the same DNA sequence.

When specifically generating sites single-strand broken in chromosome A in two chromosomes constituting homologous chromosomes, site-specific nickases that bind to the target DNA sequence in chromosome A may be designed not to bind to the corresponding DNA sequence in chromosome B. In this case, the target DNA sequence in chromosome A is a different DNA sequence from the corresponding DNA sequence in chromosome B. For example, by setting the target DNA sequence of the site-specific nickase to include the base at the specific site (different bases between homologous chromosomes) in chromosome A, the target DNA sequence becomes a DNA sequence different from the corresponding DNA sequence in chromosome B. When each of the site-specific nickases is a CRISPR-Cas system, the guide RNA may be designed to specifically bind to the target DNA sequence in chromosome A. In the case where the site-specific nickase is an artificial nuclease fused with a nickase activity-bearing enzyme, the DNA binding domain may be designed to have binding specificity to the target DNA sequence in chromosome A. In this embodiment, due to the design of the site-specific nickase, the site of single-strand break is usually within 100 bases, and more preferably within 50 bases (for example, within 40 bases, 30 bases, 20 bases, or 10 bases), from the specific site (different bases between homologous chromosomes).

In the present invention, by combining the above site-specific nucleases, single-strand breaks in homologous chromosomes can be caused in various patterns (exemplified in Figs. 2 to 5).

In the present invention, when single-strand breaks are introduced on both DNA strands in chromosome A (first and second embodiments of the present invention, Figs. 2 and 3), if the distance between the single-strand breaks is too short, double-strand breaks may occur. Therefore, the distance between single-strand break sites on different DNA strands is normally 100 bases or more, and preferably 200 bases or more.

In the present invention, the combination of site-specific nickases described above is introduced into cells. In the case of a CRISPR-Cas system, the "site-specific nickases" introduced into the cells may be, for example, in the form of a combination of a guide RNA and a Cas protein, in the form of a combination of a guide RNA and a messenger RNA translated into Cas protein, or in the form of a combination of vectors expressing them, for example. The guide RNA may be modified (such as chemical modification) to suppress degradation. In the case of an artificial nuclease fused with an enzyme having nickase activity, for example, they may be in the form of a protein, a messenger RNA translated into the protein, or in the form of a vector expressing the protein.

When employing the form of an expression vector, it includes one or more regulatory elements that are operatively bound to the DNA to be expressed. Here, "operatively bound" means that the DNA is operatively bound to the regulatory elements. Examples of the "regulatory elements" include promoters, enhancers, internal ribosome entry sites (IRES), and other expression control elements (such as transcription termination signals, for example polyadenylation signals and polyU sequences). Depending on the intended purpose, the regulatory elements may, for example, direct the constitutive expression of DNA in a variety of host cells, or may direct DNA expression only in specific cells, tissues, or organs. Further, they may direct the expression of DNA only at a specific time, or may direct the expression of artificially inducible DNA. Examples of promoters include polIII promoters (such as U6 and H1 promoters), polII promoters (such as retrovirus Rous sarcoma virus (RSV) LTR promoter, cytomegalovirus (CMV) promoter, SV40 promoter, dihydrofolate reductase promoter, β-actin promoter, phosphoglycerate kinase (PGK) promoter, and EF1α promoter), polI promoters, and combinations thereof. Those skilled in the art can select an appropriate expression vector according to the type and the like of cells for introduction.

The "cell" to which the method of the present invention is applied is not limited as long as it has a homologous chromosome, and various types of eukaryotic cells can be targeted.

Examples of "eukaryotic cells" include animal cells, plant cells, algae cells, and fungal cells. In addition to mammalian cells, examples of the animal cells include cells of fish, birds, reptiles, amphibians, and insects.

Examples of the "animal cells" include cells constituting an individual animal, cells constituting an organ-tissue extracted from an animal, cultured cells derived from an animal tissue, and the like. Specific examples include embryonic cells of embryos at various stages (such as 1-cell stage embryos, 2-cell stage embryos, 4-cell stage embryos, 8-cell stage embryos, 16-cell stage embryos, and morula stage embryos) ; stem cells such as induced pluripotent stem (iPS) cells, embryonic stem (ES) cells, and hematopoietic stem cells; and somatic cells such as fibroblasts, hematopoietic cells, neurons, muscle cells, osteocytes, hepatocytes, pancreatic cells, brain cells, and kidney cells. For the preparation of a genome-edited animal, a fertilized oocyte, that is, a fertilized egg, can be used. Particularly preferably, the fertilized egg is that of a pronuclear stage embryo. For the oocyte before fertilization, the cryopreserved one can be thawed and used.

In the present invention, "mammal" is a concept including human and non-human mammals. Examples of non-human mammals include even-toed ungulates such as cows, wild boars, pigs, sheep, and goats, odd-toed ungulates such as horses, rodents such as mice, rats, guinea pigs, hamsters, and squirrels, lagomorphs such as rabbits, and carnivores such as dogs, cats, and ferrets. The above-mentioned non-human mammal may be a domestic animal or a companion animal (pet animal), or may be a wild animal.

Examples of "plant cells" include cells of cereals, oil crops, fodder crops, fruits, and vegetables. Examples of the "plant cells" include cells constituting an individual plant, cells constituting an organ or tissue separated from a plant, cultured cells derived from a plant tissue, and the like. Examples of plant organs and tissues include leaves, stems, shoot apices (growth points), roots, tubers, root tubers, seeds, callus, and the like. Examples of plants include rice, corn, bananas, peanuts, sunflowers, tomatoes, oilseed rape, tobacco, wheat, barley, potatoes, soybeans, cotton, carnations, and the like.

In the method of the present invention (particularly, the third embodiment (Fig. 4) and the fourth embodiment (Fig. 5) of the present invention described later), it is preferable to use a cell with suppressed function of a FANC gene. FANC genes include FANCA gene and other family genes such as FANCB gene, FANCC gene, FANCD1 (BRCA2) gene, FANCD2 gene, and FANCS (BRCA1) gene. The typical base sequence of human FANCA gene and the amino acid sequence of the protein encoded by the gene are disclosed in databases (accession number: NM_000135, NP_000126), the typical base sequence of human FANCB gene and the amino acid sequence of the protein encoded by the gene are disclosed in databases (accession number: NM_001018113, NP_001018123), the typical base sequence of human FANCC gene and the amino acid sequence of the protein encoded by the gene are disclosed in databases (accession number: NM_000136, NP_000127), the typical base sequence of human FANCD1 gene and the amino acid sequence of the protein encoded by the gene are disclosed in databases (accession number: NM_000059, NP_000050), the typical base sequence of human FANCD2 gene and the amino acid sequence of the protein encoded by the gene are disclosed in databases (accession number: NM_001018115, NP_001018125), and the typical base sequence of human FANCS gene and the amino acid sequence of the protein encoded by the gene are disclosed in databases (accession number: NM_007294, NP_009225).

"Suppression of FANC gene function" in the present invention may have a mechanism that suppresses the activity of the translation product (protein) of the gene or suppresses the expression of the gene. Suppression of the activity of the translation product (protein) of the gene can be caused by introducing mutations (such as deletion, substitution, and insertion) into the gene or by inhibitor treatment, for example.

Introduction of mutations into FANC genes can be caused using genome editing systems, for example. Examples of genome editing systems include site-specific artificial nucleases targeting FANC genes, such as CRISPR-Cas (e.g., CRISPR-Cas9) systems, TALENs, and ZFNs. The action of genome editing systems cleaves genes at target sites, and mutations (base deletions, insertions, etc.) are introduced into genes through DNA repair mechanisms in cells. When making the gene deficient by introducing mutations, the deficiency may be a deficiency of the entire gene or a partial deficiency.

Examples of FANC inhibitors include molecules that inhibit the single-strand annealing activity or strand exchange activity of FANC (see, for example, Benitez, A. et al., Mol Cell 16; 71(4), 621-628 (2018) for FANC activity).

"Suppression of FANC gene function" can also be achieved by suppressing the expression of the gene. Suppression of FANC gene expression may be suppression of translation or suppression of transcription of the gene. Suppression of translation of FANC genes can be caused using double-stranded RNA (dsRNA) that binds to the transcription product of the FANC gene, for example. Examples of double-stranded RNA include siRNA, shRNA (short hairpin RNA), miRNA, etc. Suppression of transcription of FANC genes can be caused by introducing mutations into transcriptional regulatory regions of FANC genes, for example. The above-mentioned genome editing systems targeting the transcriptional regulatory regions can be used to introduce mutations. Whether or not the expression of FANC genes has been suppressed can be evaluated by immunological assay methods using antibodies that bind to translation products at the translational level, and by methods such as RT-PCR and Northern blotting at the transcriptional level.

As for molecules that suppress the function of these FANC genes, these molecules themselves may be introduced into cells, or in the case where these molecules are nucleic acids, DNA encoding the molecules (typically vectors into which the DNA is inserted) may be introduced into cells for intracellular expression.

The introduction of site-specific nickases and molecules that inhibit the function of the FANC gene into cells can be performed by a known method such as electroporation, microinjection, DEAE-dextran method, lipofection method, nanoparticle-mediated transfection method, and virus-mediated nucleic acid delivery method.

After introduction into cells, the combination of site-specific nickases causes single-strand breaks at multiple sites in the DNA region proximal to a specific site on chromosome A, and DNA containing the specific site is deleted from the chromosome. According to the present invention, such a cell with DNA deletion can be produced with high efficiency. Here, "high efficiency" means preferably 3% or more, more preferably 5% or more, and particularly preferably 10% or more (e.g., 20% or more, 300 or more, 35% or more).

The present invention also provides a kit for use in the method of the present invention, comprising a combination of the above site-specific nickases. The kit may further comprise one or more additional reagents, and examples of additional reagents include, but are not limited to, dilution buffers, reconstitution solutions, wash buffers, nucleic acid introduction reagents, protein introduction reagents, and control reagents (e.g., control guide RNAs). The kit may include instructions for use to practice the method of the present invention.

### 2. Method and kit for evaluating homologous recombination function in cells

The present invention also provides a method for evaluating homologous recombination function in cells.

In the present invention, it has been that the efficiency of deleting DNA regions is markedly improved by using cells in which the function of genes involved in homologous recombination is suppressed. Therefore, it is possible to evaluate the homologous recombination function in cells using the DNA deletion as an indicator.

In the method of the present invention, as in "1." above, into a cell having different bases between homologous chromosomes at a specific site of the homologous chromosomes consisting of chromosome A and chromosome B, a combination of site-specific nickases each of which causes a single-strand break at a DNA region proximal to the specific site is introduced, and chromosome A-specific DNA deletion generated thereby is detected.

The combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites.

It is preferable that the combination of site-specific nickases causes single-strand breaks at multiple sites in the DNA region proximal to the specific site on chromosome A, and the single-strand breaks are single-strand breaks of a single DNA strand in the proximal DNA region, and on chromosome B, the combination causes a single-strand break at one of the sites corresponding to the sites single-strand broken on chromosome A (third embodiment, Fig. 4), and the combination of site-specific nickases causes single-strand breaks at multiple sites in the DNA region proximal to the specific site on chromosome A, and the single-strand breaks are single-strand breaks of a single DNA strand in the proximal DNA region, and on chromosome B, the combination does not cause a single-strand break at one of the sites corresponding to the sites single-strand broken on chromosome A (fourth embodiment, Fig. 5).

It is also possible to practice the method for evaluating homologous recombination function in cells according to the present invention by, in homologous chromosomes consisting of chromosome A and chromosome B, causing single-strand breaks at multiple sites on the same DNA strand in chromosome A and causing single-strand breaks at multiple sites corresponding to the sites single-strand broken in chromosome A in chromosome B. In this case, single-strand breaks do not necessarily need to be introduced in the DNA region proximal to the bases different between homologous chromosomes, and can be introduced into any DNA region.

In the method of the present invention, when the frequency of chromosome A-specific DNA deletion occurring is higher than the control, the homologous recombination function in a cell is evaluated as suppressed. Here, "higher than the control" means a frequency of preferably twice or more, more preferably 5 times or more, and particularly preferably 10 times or more (e.g., 20 times) that of the control. In terms of absolute frequency, it means a frequency of preferably 4% or more, more preferably 10% or more, and particularly preferably 20% or more (e.g., 40% or more) (see Example (2) (b) and Fig. 9B). The frequency in cells in which homologous recombination function is not suppressed can be used as the control.

Suppression of homologous recombination function can occur, for example, due to suppression of the function of genes involved in homologous recombination (e.g., genetic mutations). Therefore, in cells evaluated to have suppressed homologous recombination function according to the present invention, suppression of the function of genes involved in homologous recombination is suspected. Examples of genes involved in homologous recombination include the FANC genes mentioned above.

Since suppression of homologous recombination function is known to cause cancer, the method of the present invention can target various cancer cells (e.g., breast cancer cells, ovarian cancer cells, prostate cancer cells, pancreatic cancer cells, etc.).

FANCS/FANCD1 (BRCA1/BRCA2) are responsible genes for hereditary early-onset breast and ovarian cancer, and dysfunction or decreased expression of FANCS/FANCD1 is frequently observed in cancer cells of breast and ovarian cancer patients without FANCS/FANCD1 mutations in the germline. Since dysfunction or decreased expression of FANCS/FANCD1 occurs not only due to genetic mutations but also due to expression suppression such as promoter methylation, genetic testing alone is insufficient. The present invention can evaluate whether or not homologous recombination function is suppressed regardless of the cause of suppression of homologous recombination function, and in this respect, it is superior to conventional genetic testing. On the other hand, evaluation systems using reporter genes can be used to evaluate the suppression of homologous recombination function, but it is difficult to implement such evaluation systems in patient-derived cancer cells. The method of the present invention is also superior in that it does not require a reporter gene and can directly use patient-derived cancer cells.

Currently, a test called MyChoice (Myriad), which combines measurement of the frequency of genomic changes characteristic of homologous recombination deletion and mutation testing of the above FANC genes (BRCA genes), has been put into practical use, and whether or not to use the drug (PARP inhibitor) is determined based on the results of this test. Therefore, evaluation of homologous recombination function in cancer cells by the method of the present invention can also be used to evaluate the efficacy of anticancer drugs in patients.

The present invention also provides a kit for use in the method of the present invention, comprising the combination of site-specific nickases. The kit may further include one or more additional reagents, and examples of the additional reagents include, but are not limited to, dilution buffers, reconstruction solutions, wash buffers, nucleic acid transfer reagents, protein transfer reagents, control reagents (such as control guide RNAs). The kit may include instructions for carrying out the method of the present invention.

### [Examples]

Hereinafter, the present invention is described in more detail based on Examples, but the present invention is not limited to the following Examples.

### [Materials and Methods]

### A. Materials

### (1) TK6261 cells

Lymphoblastoid cells derived from TK6 cells. They contain allele A with a loss-of-function frameshift mutation due to a 1 bp insertion in exon 4 of the thymidine kinase 1 gene (chromosome 17) and allele B with a loss-of-function frameshift mutation due to an 8 bp deletion in exon 5 and with a 1 bp insertion in exon 7 that does not affect function.

### (2) FANCA mutant TK6261 cells

Cells in which deletion mutations were generated in exon 27 of the FANCA gene in both alleles of TK6261 cells to eliminate FANCA function.

### (3) TSCER2 (TIR) cells

TK6-derived cells in which the thymidine kinase 1 (TK1) gene is compound heterozygous. The TK1 exon 4 mutation is common with TK6261 cells. TIR1 is expressed by addition of auxin and proteins with AID tags are degraded.

### (4) FANCS-protein-depleted TSCER2 (TIR) cells

TSCER2 (TIR) -FANCS^{AID/AID} cells are cells modified to knock-in auxin-inducible degron (AID) tags at the C-termini of both FANCS (BRCA1) gene alleles and express FANCS-AID. FANCS (BRCA1) protein expression can be eliminated by addition of auxin (AUX).

### (5) FANCD1-protein-depleted TSCER2 (TIR) cells

TSCER2 (TIR) -FANCD1^{AID/AID} cells are cells modified to knock-in auxin-inducible degron (AID) tags at the C-termini of both FANCD1 (BRCA2) gene alleles and express FANCD1-AID. FANCD1 (BRCA2) protein expression can be eliminated by addition of auxin (AUX).

### B. Methods

Cas9D10A mRNA (TriLink CleanCap Cas9 Nickase mRNA (5moU) - (L-7207)) with a concentration of 1.0 mg/mL in an amount of 0.9 µL, two kinds of 100 nmol/mL sgRNA, 0.45 µL each, and 2.2 µL of Neon Transfection System R buffer were mixed, and further mixed with 10 µL of TK6261 cells suspended in R buffer at 14×10⁴ cells/µL. Of this, electroporation was performed on 10 µL using Neon Transfection System under conditions of Voltage 1500V, Width 10ms, and 3 pulses.

The sgRNAs used were the following (name: target sequence. [] shows PAM sequence):
TKex4_20s: CGTCTCGGAGCAGGCAGGCG[GGG] / SEQ ID NO: 1
   S8: AGCTTCCCATCTATACCTCC[TGG] / SEQ ID NO: 2
   AS15: GAGGTAGGTTGATCTTGTGT[GGG] / SEQ ID NO: 3
   S14: TCCCACCGAAGGCCACACGC[CGG] / SEQ ID NO: 4
TKex5_mut4s: CTCGCAGAACTCCAGGGAAC[TGG] / SEQ ID NO: 5
TKex7_105s: CCCCTGGCTTTCCTGGCACT[GGG] / SEQ ID NO: 6
TKex7_mut121s: TGGCAGCCACGGCTTCCCCC[TGG] / SEQ ID NO: 7

Note that TKex4_20s is an sgRNA targeting only exon 4 on allele A, S8 is an sgRNA targeting intron 4 on both alleles, AS15 is an sgRNA targeting intron 4 on both alleles, S14 is an sgRNA targeting intron 3 on both alleles, TKex5_mut4s is an sgRNA targeting only exon 5 on allele B, TKex7_105s is an sgRNA targeting exon 7 on both alleles, and TKex7_mut121s is an sgRNA targeting only exon 7 on allele B.

Cells after electroporation were cultured overnight at 37°C, 5% CO₂ in 10% horse serum + 200 µg/ml Sodium Pyruvate/RPMI 1640 medium, then cultured for 1 week at 37°C, 5% CO₂ in 5% horse serum + 200 µg/ml Sodium Pyruvate/RPMI 1640 medium (basic medium).

Cells after culturing were sorted into 96-well plates dispensed with 200 µL of basic medium per well at 1 cell/well using FACSAria III (BD). After about 1 week of culturing, genomic DNA was extracted from 94 cloned cells derived from 1 cell using a simple DNA extraction kit Version 2 (Kaneka). Separately, genomic DNA was extracted from cultured cells without sorting using a cultured cell genomic DNA extraction kit VI (ANIMOS).

Using DNA extracted from 1-cell-derived clones as template, PCR was performed with KOD FX Neo (TOYOBO). Primer set L-AS15s was used in PCR designed so that the target sequences of sgRNA TKex4_20s, sgRNA S8 and sgRNA AS15 were included in the PCR product (full length approx. 2.1 kbp), primer set L-AS15l was used in PCR designed so that the target sequences of sgRNA TKex4_20s, sgRNA S8 and sgRNA AS15 were included in the PCR product (full length approx. 4.3 kbp), primer set L-S14 was used in PCR designed so that the target sequences of sgRNA TKex4_20s and sgRNA S14 were included in the PCR product (full length approx. 5.4 kbp), and primer set L-TKex57 was used in PCR designed so that the target sequences of sgRNA TKex5_mut4s, sgRNA TKex7_105s and sgRNA TKex7_mut121s were included in the PCR product (full length approx. 1.9 kbp). The sequences of the primers used for PCR are as follows.
L-AS15s (Forward): GCCTTTCCCATAGGTGCTAACT / SEQ ID NO: 8
L-AS15s (Reverse): AACAAAACACACTCTGGAAGATGGAACC / SEQ ID NO: 9
L-AS15l (Forward): CACAAGGCACAGGACACACTGTTAG / SEQ ID NO: 10
L-AS15l (Reverse): AACAAAACACACTCTGGAAGATGGAACC / SEQ ID NO: 11
L-S14 (Forward): GAGTACTCGGGTTCGTGAACTT / SEQ ID NO: 12
L-S14 (Reverse): GCAGCTTCCCATCTATACCTCC / SEQ ID NO: 13
L-TKex57 (Forward): AAGCCCTCACGTCTCAATAACC / SEQ ID NO: 14
L-TKex57 (Reverse): GCTTTAAGCAGACCAGTGGGTA / SEQ ID NO: 15

PCR products were electrophoresed in 0.9% TAE gel to visualize the base length of the PCR products.

Some PCR products showing deletions of several hundred bp or more were gel-purified and the DNA sequence in the exon 4 region was analyzed.

First, PCR was performed with KOD plus neo (TOYOBO) using DNA extracted from 1-cell-derived clones as template. Primer set S-EX4 was used for the 344 bp region centered on the target sequence of sgRNA TKex4_20s (exon 4 region) and primer set S-AS15 was used for the 297 bp region centered on the target sequences of sgRNA S8 and sgRNA AS15 (S8/AS15 region).
S-EX4 (Forward): TTTTCTGGACGAGGGCCTTTC / SEQ ID NO: 16
S-EX4 (Reverse): CTTCCAAGTCAGCGAGGGAAAA / SEQ ID NO: 17
S-AS15 (Forward): CCTCATGGTTCCTTTTGCTTG / SEQ ID NO: 18
S-AS15 (Reverse): GGTGGGAAAATCGCTTGAAT / SEQ ID NO: 19

Subsequently, the DNA sequences of the PCR products were analyzed by Sanger sequencing. The sequence primers were as follows.
Exon 4 region: TGAACACTGAGCCTGCTT / SEQ ID NO: 20
S8/AS15 region: CGTTTATTTTCTTGTTG / SEQ ID NO: 21

### [Examples]

### (1) Analysis of the effect of nick introduction patterns on large DNA deletions

(a) As a known method, when DNA double-strand breaks were introduced at two sites in allele A and one site in allele B using Cas9 and the combination of sgRNA TKex4_20s and sgRNA_AS15, cells with deletions of 600 bp or more occurring in only one allele were 32.3% ± 5.4% (n = 94 in triplicate) (Fig. 6C "TKex4_20s/AS15 (DSB)"). When a nick was introduced at one location on each DNA strand in allele A and one location on the antisense strand in allele B using Cas9D10A and the combination of sgRNA TKex4_20s and sgRNA_AS15, cells with deletions of 600 bp or more occurring in only one allele were 39.4% ± 2.8% (n = 94 in triplicate), which was equivalent to the method using two sites of DNA double-strand breaks (Figs. 6A(a), 6B(a), 6C "TKex4_20s/AS15 (Nick)"). On the other hand, when two tandem nicks were introduced on the sense strand in allele A and one nick was introduced on the sense strand in allele B using Cas9D10A and the combination of sgRNA TKex4_20s and sgRNA_S8, cells with deletions of 600 bp or more occurring in only one allele were 0.35% ± 0.61% (n = 94 in triplicate), showing a low frequency of deletion (Figs. 6A(b), 6B(b), 6C "TKex4_20s/S8 (Nick)"). When using nicks, it was shown that DNA deletions up to several hundred bp could be achieved with high efficiency by generating nicks on both DNA double strands.

Of 111 clones obtained using Cas9D10A as well as sgRNA TKex4_20s and sgRNA_AS15, the number of clones where only the wild-type sequence (sequence of allele B) was detected by Sanger sequencing of the exon 4 region was 100. For the remaining 11 clones, PCR was performed using primer set L-AS15s, and PCR products showing deletions of several hundred bp or more were gel-purified. DNA sequencing analysis of the exon 4 region contained in these PCR products showed that only the mutant sequence (sequence of allele A) of exon 4 was contained in all 11 clones. These results demonstrate that the deletion occurred specifically in allele A where two nicks were generated.

Sanger sequencing analysis of the exon 4 region and S8/AS15 region was performed on cells in which a deletion of >600 bp was detected in only one allele. When Cas9D10A and the combination of sgRNA TKex4_20s and sgRNA_AS15 were used, no mutations were detected at all in the exon 4 region or S8/AS15 region. On the other hand, when wild-type Cas9 and the combination of sgRNA TKex4_20s and sgRNA_AS15 were used, mutations were detected in 4.44 ± 1.84% of cells in the exon 4 region and 100 ± 0.0% of cells in the S8/AS15 region. These results demonstrate that with DSBs, it is very difficult to normally maintain the allele without deletion, while with nicks, accuracy can be maintained very highly.

(b) When two tandem nicks were introduced on the sense strand in allele A and one nick was introduced on the sense strand in allele B in TK6261 cells using Cas9D10A and the combination of sgRNA TKex5_mut4s and sgRNA TKex7_105s, cells with deletions of 500 bp or more occurring in only one allele were 0.71 ± 0.61% (n = 94 in triplicate) (Figs. 7A(a), 7B "TKex5_mut4s/TKex7_105s"). On the other hand, when two tandem nicks were introduced on the sense strand in allele A and no nicks were introduced in allele B in TK6261 cells using Cas9D10A and the combination of sgRNA TKex5_mut4s and sgRNA TKex7_mut121s, cells with deletions of 500 bp or more occurring in only one allele reached 2.84 ± 1.62% (n = 94 in triplicate) (Figs. 7A(b), 7B "TKex5_mut4s/TKex7_mut121s"). This demonstrates that when tandem nicks are introduced in allele A, avoiding the introduction of nicks in allele B can significantly improve the frequency of long deletions occurring between the nicks.

### (2) Analysis of the effect of FANC gene function suppression

(a) When two tandem nicks were introduced on the sense strand in allele A and one nick was introduced on the sense strand in allele B in FANCA-knockout TK6261 cells using Cas9D10A and the combination of sgRNA TKex4_20s and sgRNA_S14, cells with deletions of 1,000 bp or more occurring in only one allele reached 15.2% ± 2.68% (n = 94 in triplicate) (Figs. 8A, 8B(a), 8C "FANCA Deficient"). On the other hand, in FANCA-expressing TK6261 cells (parent strain), cells with deletions of 1,000 bp or more occurring were only 1.42% ± 0.61% (n = 94 in triplicate) (Figs. 8A, 8B(b), 8C "TK6261"). This demonstrates that suppression of FANCA function can significantly improve the frequency of long deletions between nicks.
(b) Nicks were introduced in the same way as (a) into TSCER2 (TIR) cells (TSCER2 (TIR) -FANCS^{AID/AID}) capable of depleting FANCS protein by auxin induction and TSCER2 (TIR) cells (TSCER2 (TIR) -FANCD1^{AID/AID}) capable of depleting FANCD1 protein by auxin induction. As a result, cells with deletions of 1,000 bp or more occurring in only one allele were 45.4% ± 1.6% in FANCS-protein-depleted cells and 47.5% ± 11.3% in FANCD1-protein-depleted cells (n = 94 in triplicate) (Fig. 9A, 9B). Note that when auxin was not added and FANCS protein was not depleted, the value was 1.77% ± 1.23%, and when auxin was not added and FANCD1 protein was not depleted, the value was 2.12% ± 1.06% (n = 94 in triplicate). This demonstrates that as with FANCA, suppression of FANCS and FANCD1 function can significantly improve the frequency of long deletions between nicks.

### [Industrial Applicability]

As described above, according to the present invention, by utilizing single-strand breaks by site-specific nickases, DNA containing a specific site (different bases between homologous chromosomes) can be deleted in one of the homologous chromosomes. Since the present invention does not use double-strand breaks or exogenous donor DNA, it can greatly contribute to gene therapy for diseases caused by hetero mutations owing to its high safety. Further, according to the present invention, homologous recombination function in cells can be evaluated using the DNA deletion specific to one of the homologous chromosomes as an indicator, which can also greatly contribute to the diagnosis of diseases such as cancer.

## Claims

1. A method for producing a cell having a DNA deletion specific to chromosome A of homologous chromosomes consisting of chromosome A and chromosome B, comprising:
introducing, into a cell having different bases between homologous chromosomes at a specific site of the homologous chromosomes, a combination of site-specific nickases each of which causes a single-strand break at a DNA region proximal to the specific site, wherein
the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites.

2. The method according to claim 1, wherein the cell having different bases between homologous chromosomes at a specific site of the homologous chromosomes is a cell with suppressed function of a FANC gene.

3. The method according to claim 1 or 2, wherein the bases different between homologous chromosomes are bases at a mutation site.

4. The method according to any one of claims 1 to 3, wherein each of the site-specific nickases is a CRISPR-Cas system.

5. A kit for use in the method according to claim 1, comprising:
in a cell having different bases between homologous chromosomes at a specific site of the homologous chromosomes, a combination of site-specific nickases each of which causes a single-strand break at a DNA region proximal to the specific site, wherein
the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites.

6. The kit according to claim 5, wherein the cell having different bases between homologous chromosomes at a specific site of the homologous chromosomes is a cell with suppressed function of a FANC gene.

7. A method for evaluating homologous recombination function in a cell, comprising:
introducing, into a cell having different bases between homologous chromosomes at a specific site of the homologous chromosomes consisting of chromosome A and chromosome B, a combination of site-specific nickases each of which causes a single-strand break at a DNA region proximal to the specific site; and detecting chromosome A-specific DNA deletion generated thereby, wherein
the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites, and
the homologous recombination function in a cell is evaluated as suppressed when a frequency of chromosome A-specific DNA deletion occurring is higher than a control.

8. The method according to claim 7, wherein the cell is a cancer cell.

9. The method according to claim 7 or 8, wherein each of the site-specific nickases is a CRISPR-Cas system.

10. A kit for use in the method according to claim 7, comprising:
in a cell having different bases between homologous chromosomes at a specific site of the homologous chromosomes consisting of chromosome A and chromosome B, a combination of site-specific nickases each of which causes a single-strand break at a DNA region proximal to the specific site, wherein
the combination of site-specific nickases causes single-strand breaks at multiple sites in the DNA region proximal to the specific site on chromosome A, and the single-strand breaks are single-strand breaks of both DNA strands or a single DNA strand in the proximal DNA region, and on chromosome B, the combination either causes a single-strand break at one of the sites corresponding to the sites single-strand broken on chromosome A, or does not cause single-strand breaks at the corresponding sites.
